Europäisches Patentamt

European Patent Office

Office européen des brevets

(11) Publication number : **0 506 402 A2**

# EUROPEAN PATENT APPLICATION

(21) Application number : **92302631.4**

(51) Int. Cl.$^5$ : **C07C 4/04,** C10G 9/16

(22) Date of filing : **26.03.92**

(30) Priority : **27.03.91 US 676043**
**27.03.91 US 676044**

(43) Date of publication of application :
**30.09.92 Bulletin 92/40**

(84) Designated Contracting States :
**BE DE ES FR GB IT NL PT**

(71) Applicant : **BETZ EUROPE, INC.**
**Somerton Road**
**Trevose, PA 19053-6783 (US)**

(72) Inventor : **Reid, Dwight Kendall**
**10819 Willomist Drive**
**Houston, TX 77064 (US)**
Inventor : **Fields, Daniel Eugene**
**209 Walnut, Apt. A**
**Metairie, LA 70005 (US)**

(74) Representative : **W.P. Thompson & Co.**
**Coopers Building, Church Street**
**Liverpool L1 3AB (GB)**

(54) Inhibition of coke formation.

(57) Methods and compositions are provided for inhibiting the formation and deposition of pyrolytic coke on metal surfaces in contact with a hydrocarbon feedstock undergoing pyrolytic processing. Coke inhibition is achieved by adding a combination of a boron compound and a dihydroxybenzene compound. Dihydroxybenzene compounds also proved effective when added by themselves to the hydrocarbon feedstock.

EP 0 506 402 A2

The present invention is directed towards compositions and methods for inhibiting the formation and deposition of coke on metallic surfaces in contact with hydrocarbon feedstock which is undergoing high temperature pyrolytic processing. The compositions and methods employ, a dihydroxybenzene compound, preferably in conjunction with a boron compound to retard coke formation and deposition on metal surfaces in contact with the hydrocarbon which are in excess of 760°C (1400°F), usually in excess of 871°C (1600°F).

Coke deposition is generally experienced when hydrocarbon liquids and vapours contact the hot metal surfaces of petroleum processing equipment. The complex makeup of the hydrocarbons at elevated temperatures and contact with hot metal surfaces makes it unclear what changes occur in the hydrocarbons. It is thought that the hydrocarbons undergo various changes through either chemical reactions and/or the decomposition of various unstable components of the hydrocarbons. The undesired products of these changes in many instances include coke, polymerized products, deposited impurities and the like. Regardless of the undesired product that is produced, reduced economies of the process is the result. If these deposited impurities remain unchecked, heat transfer, throughput and overall productivity are detrimentally effected. Moreover, downtime is likely to be encountered due to the necessity of either replacing the affected parts or cleaning the fouled parts of the processing system.

While the formation and type of undesired products is dependent on the type of hydrocarbon being processed and the operating conditions of the processing, it may generally be stated that such undesired products can be produced at temperatures as low as 38°C (100°F). However, the undesired products are much more prone to formation as the temperature of the processing system and the metal surfaces thereof in contact with the hydrocarbon increase. At these higher temperatures, coke formation is likely to be produced regardless of the type of hydrocarbon being charged. The type of coke formed, be it amorphous, filamentous or pyrolytic, may vary somewhat; however, the probability of coke formation is quite high.

Coke formation also erodes the metal of the system in two ways. The formation of catalytic coke causes the metal catalyst particle to become dislodged. This results in rapid metal loss and ultimately metal failure. The other erosive effect occurs when carbon particles enter the hydrocarbon stream and act as abrasives on the tube walls of the processing system.

As indicated in US-A- 4 962 264, coke formation and deposition are common problems in ethylene (olefin) plants which operate at temperatures of the metal surfaces are sometimes at 871°C (1600°F) and above. The problem is prevalent in the cracking furnace coils as well as in the transfer line exchangers (TLEs) where pyrolytic type coke formation and deposition is commonly encountered. Ethylene plants originally produced simple olefins such as ethylene, propylene, butene and butadiene from a feed of ethane, propane, butane and mixtures thereof. Later developments in this area of technology have led to the cracking of even heavier feedstocks to produce aromatics and pyrolysis gasoline as well as the light molecular weight olefins. Feed stocks now include kerosene light naphtha, heavy naphtha and gas oil. According to the thermal cracking processes utilized in olefin plants, the feedstocks are generally cracked in the presence of steam in tubular pyrolysis furnaces. The feedstock is preheated, diluted with steam and this mixture is then heated in the pyrolysis furnace to about 816°C (1500°F) and above, most often in the range of 816°C to 899°C (1500°F to 1650°F).

The effluent from the furnace is rapidly quenched by direct means or in exchangers which are designed to generate steam at pressures of 2859 to 5617 kPa absolute (400 tc 800) psig. This rapid quench reduces the loss of olefins by minimizing any secondary reactions. The cooled gas then passes to a prefractionator where it is cooled by circulating oil streams to remove the fuel oil fraction. In some designs, the gas leaving the oil is further cooled with oil before entering the prefractionator. In either case, the heat transferred to the circulating oil stream is used both to generate steam and to heat other process streams. The mixture of gas and steam leaving the prefractionator is further cooled in order to condense the steam and most of the gasoline product in order to provide reflux for the prefractionator. Either a direct water quench or heat exchangers are used for this post prefractionator cooling duty.

After cooling, cracked gas at, or close to atmospheric pressure, is compressed in a multistage compression system to much higher pressures. There are usually four or five stages of compression with interstage cooling and condensate separation between stages. Most plants have hydrocarbon condensate stripping facilities. Condensate from the interstage knockout drum is fed to a stripper when the $C_2$ and lighter hydrocarbons are separated. The heavier hydrocarbons are fed to the depropanizer.

Accordingly, there is a need in the art to inhibit the formation and deposition of coke on surfaces in contact with high temperature hydrocarbons to improve the efficiencies of the processing system. Moreover, there is a particular need to retard coke formation and deposition during the high temperature pyrolysis and cracking of hydrocarbons.

FR- 2 202 930 (Chem, Abst. Vol. 83:30687K) is directed to tubular furnace cracking of hydrocarbons where molten oxides or salts of Group III IV or VIII metals (e.g., molten lead containing a mixture of $K_3VO_4$, $SiO_2$ and NiO) are added to a pretested charge of, for example, naphtha steam at 500°C 932°F. This treatment is stated as having reduced deposit and coke formation in the cracking section of the furnace.

Starshov et al., Izv. Vyssh. Uchebn. Zaved Neft Gaz, 1977 (Chem. Abst. 87:154474r) describes the pyrolysis of hydrocarbons in the presence of aqueous solutions of boric acid. Carbon deposits were minimized by this process.

Nokonov et al., U.S.S.R. No. 834,107, 1981; (Chem Abst. 95:13565v) describes the pyrolytic production of olefins with peroxides present in a reactor, the internal surfaces of which have been pretreated with an aqueous alcoholic solution of boric acid. Coke formation is not mentioned in this patent since the function of boric acid is to coat the inner surface of the reactor and thus decrease the scavenging of peroxide radicals by the reactor surface.

Starshov et al., Neftekhimiya 1979 (Chem. Abst. 92:8645j) describes the effect of certain elements including boron on coke formation during the pyrolysis of hydrocarbons to produce olefins.

US-A- 3 531 394 (Koszman) teaches the inhibition of carbon formation in the thermal cracking of petroleum fractions. His process teaches the use of bismuth and phosphorus containing compounds to reduce carbon formation.

US-A- 3 661 820 (Foreman et al.) teaches a composition that is used as a coating for steel surfaces. This composition will prevent carburization in gas carburizing, pack carburizing and carbonitriding mediums. The composition taught is a boron compound selected from boric acid, boric oxide and borax; water soluble organic resin; carrier fluid of water and thickening and drying agents.

US-A- 2 063 596 (Feiler) teaches a method of treating the metal of a system processing hydrocarbons at high temperatures. This patent discloses the suppression of the deposition of carbon on the metal surfaces of a hydrocarbon process using the metals tin, lead, molybdenum, tungsten and chromium to coat the metal surfaces. This patent conjectures as to the use of a metalloid of boron as a treating agent.

GB-A- 296,752 teaches a method of preventing deposition of coke or soot on metal surfaces in contact with hydrocarbons at high temperatures. The metals are treated directly with metalloids of boron, arsenic, bismuth, antimony, phosphorus or selenium.

GB-A- 275,662 teaches a process for preventing the formation of carbon monoxide in a hydrocarbon cracking operation. This process involves coating the metal surfaces that contact the hydrocarbon with metalloids of boron, arsenic, antimony, silicon, bismuth, phosphorus or selenium.

US-A- 1 847 095 (Mittasch et al.) teaches a process for preventing the formation and deposition of carbon and soot in hydrocarbon processes operating at elevated temperatures. This process consists of adding to the hydrocarbon stream hydrides of metalloids selected from the group of boron, arsenic, antimony bismuth, phosphorus, selenium and silicon.

US-A- 3 687 840 (Sze et al.) teaches a method of stopping plugs in a delayed coker unit that result from the formation and deposition of coke. This process employs sulphur and sulphur compounds as the inhibiting agents.

US-A- 4 555 326 (Reid) teaches a method of inhibiting the formation and deposition of filamentous coke in hydrocarbon processing systems operating at high temperatures. The metal that contacts the hydrocarbon fluid is first treated ("boronized") by contacting it with boron, boron oxide compounds or metal borides.

US-A- 4 724 064 (Reid) teaches a method of inhibiting the formation and deposition of filamentous coke on metal surfaces in contact with a hydrocarbon fluid at high temperatures. Boron oxide compounds, metal borides and boric acid which is substantially free of water are the inhibiting agents.

US-A- 4 680 421 (Forester et al.) discloses a method of inhibiting the formation and deposition of pyrolytic coke on the heated metal surfaces of a pyrolysis furnace. This method employs an ammonium borate compound to inhibit the deposition on the 871°C (1600°F) and higher temperature metal surfaces.

US-A- 3 342 723 (Godar) teaches a method of inhibiting the formation and deposition of coke-like deposits and soft sludges on structural surfaces in contact with a hydrocarbon undergoing petroleum refining. This method utilizes an ortho substituted aromatic compound or substituted monocyclic compound such as catechol as the antifouling agent.

The present invention pertains to compositions and methods for inhibiting the formation and deposition of pyrolytic coke on the heated metal surfaces in contact with a hydrocarbon feedstock which is undergoing pyrolytic processing to produce lower hydrocarbon iractions and said metal surfaces having a temperature of about 760°C (1400°F) or above, usually about 871°C (1600°F) or above.

While the invention is applicable to any system where coke is produced, this invention is surprisingly effective during the high temperature pyrolysis and cracking of a hydrocarbon feedstock.

According to the present invention there is provided a method of inhibiting the formation and deposition of pyrolytic coke on the heated metallic surfaces in contact with a hydrocarbon feedstock which is undergoing pyrolytic processing to produce lower hydrocarbon fractions and the metallic surfaces have a temperature of about 760°C (1400°F) or higher, which comprises adding to the hydrocarbon feedstock being pyrolytically processed a dihydroxybenzene compound.

According to the present invention there is also provided a method for inhibiting the formation and deposition of pyrolytic coke on the heated metallic surfaces in contact with a hydrocarbon feedstock which is undergoing pyrolytic processing to produce lower hydrocarbon fractions and the metallic surfaces have a temperature of about 760°C (1400°F) or higher, which comprises adding to the hydrocarbon feedstock being pyrolytically processed a synergistic combination of a boron compound and a dihydroxybenzene compound.

According to the present invention there is further provided a composition for inhibiting the formation and deposition of pyrolytic coke which comprises a boron compound and a dihydroxybenzene compound.

The compositions and methods of this invention are surprisingly effective coke retardants at the high temperatures of the metal surfaces of the pyrolytic furnace, reaching temperatures of 760°C (1400°F) usually 871°C (1600°F) and above, and up to 1121°C (2050°F). These temperature are commonly encountered in olefin plants where hydrocarbon feedstocks containing ethane, propane, butane, light naphtha, heavy naphtha, gas oil, and mixtures of the same are cracked to produce lower and/or olefinic hydrocarbon fractions. Coking is a significant problem for if it is left untreated, the operation will eventually shut down.

In these pyrolysis systems, the components of the pyrolytic furnace, as well as the ancillary parts are composed of ferrous metal. Iron, as well as iron alloys such as low and high carbon steel, and nickel-chromium-iron alloys are customarily used for the production of hydrocarbon and petroleum processing equipment such as furnaces, transmission lines, reactors, drums, heat exchangers, fractionators, and the like.

It has been found that during the high temperature pyrolytic processing of hydrocarbons coke will form and deposit on the stainless steel surfaces of the system. This formation and deposition on the stainless steel surfaces can be significantly reduced in accord with the test herein by use of a composition of a boron compound and a dihydroxybenzene compound, specifically ammonium biborate and hydroquinone.

Accordingly, coke formation may also be reduced on iron, chromium and nickel based metallurgical surfaces in contact with pyrolysis products in high temperature pyrolytic furnaces.

The boron compounds are effective when formulated with glycollic-type solvents, in particular ethylene glycol, propylene glycol, glycerol, hexlene glycols and polyethylene glycols.

Boron oxide, ammonium pentaborate and sodium borate may be used as effective boron compounds in the present invention.

The dihydroxybenzene compounds are effective when formulated in water with a co-solvent such as, for example, butyl carbitol or ethylene glycol.

Resorcinol, catechol, 1, 2-naphthoquinone, 1,4-naphthoquinone, 1,4-naphthoquinone and 4-tert-butyl-resorcinol may be effective dihydroxybenzene compounds in the present invention.

The boron compounds and dihydroxybenzene compounds are formulated separately. The mixtures can then be added directly to the hydrocarbon feedstock or charge before and/or during the pyrolytic processing, or the treatment composition may be mixed with steam carried to the cracking zone in accordance with conventional cracking techniques.

The treatment dosages for the boron compounds and the dihydroxybenzene compounds are dependent upon the severity of the coking problem, location of such problem, and the amount of active compound in the formulated product. For this reason, the success of the treatment is totally dependent upon the use of a sufficient amount of the treatment composition thereby to effectively inhibit coke formation and deposition.

Preferably, the total amount of boron compound added is from about 1 ppm to about 2500 ppm per million parts of feedstock. The dihydroxybenzene compound added is from about 1 ppm to about 2500 ppm per million parts of feedstock when used alone or in combination.

More preferably, the boron compound ranges from about 10 ppm to about 250 ppm and the dihydroxybenzene from about 20 ppm to about 500 ppm per million parts of feedstock.

The preferred weight ratio of dihydroxybenzene compound to the boron compound ranges from 1:1 to 4:1, most preferably 2.6:1. The preferred embodiment employs a 35 weight percent ammonium biborate in ethylene glycol and a 20 weight percent hydroquinone in ethylene glycol combination.

The invention will now be further described with reference to a number of specific Examples which are to be regarded solely as illustrative, and not as restricting the scope of the invention.


**EXAMPLES**


In order to establish the efficacy of the invention, various tests were conducted using a propane feedstock with dilution steam added to enhance cracking. The apparatus and procedure used for the testing were are follows:

**Apparatus**

The high temperature fouling apparatus (HTFA) consists of five subsections which together simulate the pyrolysis of gaseous hydrocarbons to make the light olefinic end products and the undesirable by-product, coke, that is formed on the heated metal surfaces during the pyrolysis reaction.

The feed preheat section is built of 316 stainless steel tubing and fittings and allows the mixing of nitrogen or oxygen containing gas with steam during the start up and shut down of the HFTA and the propane with steam during the actual test. Steam is supplied at 377 kPa absolute (40 psig) by a steam generator and nitrogen, oxygen containing gas, or propane is fed from compressed gas cylinders. The gases and steam are heated to about 149°C (300°F) at which point small amounts of water (blank test) or candidate material is slowly injected into the stream by a syringe pump. The gases/candidate material are further preheated to about 260°C (500°F) before flowing through a 3.96 metre (13-foot) long coiled 316 SS tube inside an electrically heated furnace. The gases are heated at a furnace temperature of approximately 87°C (188°F) and exit the furnace at 621°C-788°C (1150°F - 1450°F).

Following the furnace tube, the gases travel through the coker rod assembly. This consists of a 316 SS rod which is electrically heated to 816°C (1500°F) while the gases flow around the heated rod inside a 316 SS shell. The rod is electrically heated through a silicon controlled rectifier (SCR), then through two 4 to 1 stepdown transformers in series to achieve low voltage (3-4 volts) and high amperage (200 amps) heating of the rod. A temperature controller is used to achieve power control through the SCR to obtain a 816°C (1500°F) rod temperature.

Upon exiting the coker rod, the gases pass through a condenser coil and then through three knockout flasks in ice baths to remove the water (steam) from the product gases. The small amount of remaining entrained water vapour in the gases is removed by passing through drierite granules.

The specific gravity of the product gas is determined in a gas densitometer and the gases are analyzed using gas chromatography to determine yields. The remaining gases are vented through a safety hood exhaust.

**Test Procedure**

The furnace was turned on and the temperature thereof was stabilized at 704°C (1300°F) while feeding nitrogen and steam. The coker rod was heated to 816°C (1500°F). The nitrogen was replaced with oxygen containing gas (air) and furnace temperatures were then slowly increased to 816°C (1500°F) over a period of ten minutes. Then the air was replaced with nitrogen and the coke inhibitor or water (blank), as the case may be, was injected into the mixed gas or steam line at about 149°C (300°F) gas temperature while the furnace temperature was slowly raised to 1027°C (1880°F) over 20-25 minutes.

Then the nitrogen feed was gradually switched to propane feed over about 5 minutes. The temperature of the furnace dropped due to the propane cracking reaction and was allowed to increase to the maximum attainable furnace temperature 1027°C (1880°F or less) over approximately a 30 minute period. The product gases were analyzed by gas chromatography and the temperatures, flowrates, pressures and product gas gravity recorded every 35 minutes during the 160 minute test on propane/steam feed. Gases exit the furnace tube at about 621°C - 788°C (1150°F - 1450°F) and exit the coker shell at about 524°C - 538°C (975°F - 1000°F) temperatures.

During a normal 160 minute run, approximately 3200-3300 grams of propane were fed and 1000-2000 grams of steam fed (determined from the condensate collected) for hydrocarbon to steam rates of about 1.6:1 to 3.2:1. Following shutdown and cooling, the furnace tube and coker shell were cleaned and the coke collected and weighed. The collected coke was then burned in air at 760°C (1400°F) for one hour and the residue remaining weighed and termed gray matter (corrosion products from furnace tube).

Table 1 reports the results of the above test by indicating the amount of coke formed for various antifoulants. A high percentage coke reduction value is indicative of effective treatment.

## TABLE I

High temperature fouling apparatus (HFTA)

Results for coke inhibiting compounds

704°C-816°C (1300°-1500°F) furnace steam/air decoke

816°C-1021°C (1500°F-1870°F) furnace antifoulant/ $N_2$/steam

1021°C (1870°F) furnace propane (14.16 $\ell$/min (0.5SCFM))/steam/antifoulant for 160 minutes

| Additive | No. of Runs | Average %<br>Coke Reduction |
|---|---|---|
| Blank | 18 | -3 |
| 10% HQ/23.33%<br>AmBiBor in EG | 5 | 70 |

HQ = Hydroquinone

AmBiBor = Ammonium Biborate

EG = Ethylene Glycol

The inventive composition was evaluated as a pretreatment agent to determine the amount of coke deposited. 20ml of the treating agent was injected into the steam line of the HFTA over two hours and allowed to flow through the furnace tube and coker rod heated to 816°C (1500°F). following this pretreatment, the tube and rod were removed and weighed. The tube and the rod were then reassembled and a blank propane/stem run was conducted on the pretreated surfaces. The results of these pretreated HFTA tests are shown in Table II.

TABLE II

High temperature fouling apparatus (HFTA)

Results for coke inhibiting compounds

2 hour pretreatment at 816°C-1021°C (1500°-1870°F)

furnace propane 14.16 $\ell$/min (0.5 SCFM))/stream for

160 minutes

| Additive | metal (ppm) | Coke Level (Grams) |
|---|---|---|
| Blank | | 3.05, 2.14, 1.11<br>2.1 avg. |
| 10% HQ/23.33%<br>AmBiBor in EG | 608 HQ<br>223 B | 0.34 |

HQ = Hydroquinone

AmBiBor = Ammonium Biborate

EG = Ethylene Glycol

The results of Table II indicate that the inventive composition is effective at inhibiting the deposition of coke in pyrolytic furnaces both as a pretreatment agent and as a treatment agent during hydrocarbon processing.

The following data was generated by employing 310 stainless less steel furnace tube and coker rod. The coke formed during the propane/steam/antifoulant run was burned off and the levels of CO and $CO_2$ was monitored. These results appear in Tables III and IV.

## TABLE III

### High Temperature Fouling Apparatus (HTFA)

$1021^\circ$C ($1870^\circ$F) Furnace, Propane 14.16 $\ell$/min (0.5 SCFM))/Steam/
Antifoulant 310 Stainless Steel Metallurgy Furnace Tube and Coker Rod

| Run No. | Additive (ppm) in EG | Steam Rate (ml/min) | Time on Propane (min) | Coke Value [1] | Predicted Coke Value [2] | % Change in Coking vs. Predicted [3] |
|---|---|---|---|---|---|---|
| 1 | Blank | 8.95 | 279 | 2.01 | 2.48 | - 19 |
| 4 | Blank | 7.34 | 300 | 3.30 | 2.69 | 23 |
| 9 | Blank | 7.28 | 300 | 3.91 | 3.70 | 6 |
| 14 | Blank | 6.58 | 316 | 4.28 | 4.56 | - 6 |
| 3 | HQ(240) AmBiBor(92) | 7.06 | 294 | 1.73 | 2.41 | - 28 |
| 6 | HQ(457) AmBiBor(25) | 6.13 | 300 | 2.58 | 2.79 | - 7 |
| 7 | HQ(443) AmBiBor(24) | 6.27 | 234 | 2.35 | 3.03 | - 23 |
| 8 | HQ(237) AmBiBor(91) | 6.97 | 300 | 2.89 | 3.42 | - 15 |
| 13 | HQ(410) AmBiBor(23) | 7.00 | 332 | 2.89 | 4.46 | - 35 |
| 10 | HQ(585) | 7.60 | 301 | 5.90 | 3.99 | 48 |

### $CO_2$ and CO Measurements

| Run No. | $CO_2$ Area [4] | CO Area [4] | Resid. Coke gm. |
|---|---|---|---|
| 1 | 4.4 | 0.39 | 0.37 |
| 4 | 8.6 | 2.04 | 0.08 |
| 9 | 14 | 6.8 | 3.85 |
| 14 | 6.8 | 3.85 | 0.77 |
| 3 | 5.7 | 0.27 | 0.06 |
| 6 | 7.0 | 1.39 | 0.08 |
| 7 | 6.2 | 1.30 | 0.09 |
| 8 | 8.1 | 1.41 | 0.07 |
| 13 | 8.0 | 1.00 | 0.29 |
| 10 | 11.9 | 6.03 | 0.06 |

[1] Coke value = $CO_2$ * 0.273 + CO x 0.429 + coke resid.

[2] Predicted coke value = 0.206 x Run No. + 0.254 x steam rate

[3] % Change in coking = [(Coke Value - Predicted Coke value)/ predicted coke value ] x 100

4 Area below the peak on a Gas Chromatography Trace

HQ = Hydroquinone

Ambibor = Ammonium Biborate

EG = Ethylene Glycol


## TABLE IV

### High Temperature Fouling Apparatus (HTFA)
$1021°C$ ($1870°F$) Furnace, Propane 14.16 $\ell$/min (0.5 SCFM))/Steam/
Antifoulant Inconel 800 Metallurgy Furnace Tube

| Run No. | Additive (ppm) in EG | Steam Rate (ml/min) | Time on Propane (min) | [1] Coke Value | [2] Predicted Coke Value | [3] % Change in Coking vs. Predicted |
|---|---|---|---|---|---|---|
| 1 | Blank | 7.54 | 300 | 8.86 | 8.0 | 11 |
| 4 | Blank | 6.08 | 300 | 10.10 | 12.2 | - 17 |
| 8 | Blank | 6.81 | 271 | 21.00 | 20.0 | 5 |
| 5 | HQ(213) AmBiBor(82) | 7.31 | 300 | 5.06 | 15.0 | - 66 |
| 6 | HQ(211) AmBiBor(81) | 7.04 | 298 | 9.70 | 16.6 | - 42 |
| 11 | HQ(387) AmBiBor(148) | 7.11 | 310 | 4.75 | 25.7 | - 82 |
| 7 | HQ(632) | 6.52 | 296 | 34.71 | 18.0 | - 93 |
| 2 | AmBiBor(120) | 7.60 | 300 | 23.56 | 9.9 | 139 |
| 3 | AmBiBor(62) | 7.59 | 298 | 31.60 | 11.7 | 171 |
| 9 | AmBiBor(209) | 6.80 | 304 | 1.22 | 21.8 | - 94 |
| 10 | AmBiBor(51) | 7.20 | 300 | 18.06 | 24.0 | - 25 |

CO$_2$ and CO Measurements

| Run No. | CO$_2$ Area [4] | CO Area [4] | Resid. Coke gm. |
|---|---|---|---|
| 1 | 17.67 | 3.39 | 2.59 |
| 4 | 22.80 | 5.97 | 1.32 |
| 8 | 48.81 | 12.36 | 2.39 |
| 5 | 10.66 | 4.07 | 0.41 |
| 6 | 18.58 | 7.73 | 1.32 |
| 11 | 7.22 | 4.07 | 0.15 |
| 7 | 79.40 | 24.52 | 2.55 |
| 2 | 61.43 | 12.50 | 1.45 |
| 3 | 80.30 | 18.42 | 1.80 |
| 9 | 2.44 | 0.85 | 0.19 |
| 10 | 41.52 | 13.12 | 1.11 |

[1] Coke value = CO$_2$ x 0.273 + CO x 0.429 + resid coke.

[2] Predicted coke value = 1.81 x Run Number + 0.82 x steam rate

[3] % Change in coking = [(Coke Value - Predicted Coke value)/

predicted coke value ] x 100

4 Area below the peak on a Gas Chromatography Trace

HQ = Hydroquinone
AmBiBor = Ammonium Biborate
EG = Ethylene Glycol

As seen in Tables III and IV, the inventive composition reduced coke formation by 21.6% and 63.3% respectively. Hydroquinone and ammonium biborate when employed by themselves were less effective.

Accordingly, from the above, it is clear that a combination of hydroquinone and ammonium biborate is effective as a coke retarding treatment under the simulated pyrolysis conditions above noted.

Table V reports the results of the above test by indicating the amount of coke formed for various antifoulants. A high percentage coke reduction value is indicative of effective treatment. This testing was performed utilizing only the dihydroxybenzene compound.

TABLE V

High Temperature Fouling Apparatus (HFTA)
Results for coke inhibiting compounds
704°C-816°C (1300°F-1500°F) Furnace Steam/Air Decoke
816°C-1021°C (1500°F-1870°F) Furnace Propane (14.16 $\ell$/min (0.5 SCFM))/
Steam/Antifoulant for 2.67 Hrs.

| Antifoulant (ppm.Metal) | Steam (ml/min) | Coke Formed (Grams) | Gray. Formed[1] (Grams) | Predicted[2] Coke.Grams | % Coke[3] Reduction |
|---|---|---|---|---|---|
| 10% HQ/EG (176) | 10.64 | 0.57 | 5.53 | 0.60 | 4 |
| 10% HQ/EG (168) | 10.91 | 0.06 | 5.46 | 0.58 | 90 |
| 10% HQ/EG (173) | 12.88 | 0.44 | 0.75 | 0.49 | 11 |
| 30% HQ/EG (561) | 11.62 | 0.30 | 0.53 | 0.55 | 45 |
| 20% HQ/EG (229) | 14.27 | 0.22 | 0.18 | 0.45 | 51 |
| 20% HQ/EG (261) | 9.56 | 0.31 | 0.26 | 0.67 | 54 |
| 10% 1-NAPH/EG (170) | 13.66 | 0.78 | 1.28 | 0.47 | - 67 |
| 10% t-BUT HQ/EG (193) | 9.50 | 0.81 | 3.31 | 0.67 | - 21 |
| 10% 1,5-DHN/EG (171) | 13.63 | 1.05 | 5.35 | 0.47 | -125 |

[1] Gray is the corrosion products removed from furnace tube/coker shell that remain after the total coke collected is burned at 1400°F in air.

[2] Predicted coke = 6.37/condensate rate (ml/min)

[3] % Coke Reduction = [1 - coke formed/predicted coke] * 100

| | |
|---|---|
| HQ | = Hydroquinone |
| EG | = Ethylene Glycol |
| EA | = Ethanolamine |
| 1-NAPH | = 1-Naphthol |
| t-BUT HQ | = Tertbutyl Hydroquinone |
| 1,5-DHN | = 1,5-Dihydroxynaphthalene |

The six HFTA runs treated with HQ in solvents exhibited an average coke reduction of 43% +/-31%. Employing the Mann-Whitney statistical comparison test, the treated runs were greater than the untreated runs at a 95.2% confidence level.

Accordingly, from the above, it is clear that dihydroxybenzene compounds, particularly hydroquinone, are effective as coke retarding treatments under the simulated pyrolysis conditions above noted.

**Claims**

1. A method of inhibiting the formation and deposition of pyrolytic coke on the heated metallic surfaces in contact with a hydrocarbon feedstock which is undergoing pyrolytic processing to produce lower hydrocarbon fractions and the metallic surfaces have a temperature of about 760°C (1400°F) or higher, which comprises adding to the hydrocarbon feedstock being pyrolytically processed a dihydroxybenzene compound.

2. A method according to claim 1, wherein the dihydroxybenzene compound is selected from hydroquinone, catechol, resorcinol, 1,2-naphthoquinone, 1,4-naphthoquinone and 4-tert-butylresorcinol.

3. A method according to claim 1 or 2, wherein the dihydroxybenzene compound is contained in an ethylene glycol:water co-solvent carrier or a butyl carbitol:water co-solvent carrier.

4. A method according to any of claims 1 to 3, wherein the dihydroxybenzene compound is added to the hydrocarbon feedstock from about 1 part per million to about 2500 parts per million parts of hydrocarbon feedstock.

5. A method according to any of claims 1 to 4, which is performed in a pyrolysis furnace.

6. A method according to any of claims 1 to 4, which is performed in a delayed coker system.

7. A method according to any of claims 1 to 6, wherein the metallic surfaces are ferrous metal surfaces.

8. A method according to any of claims 1 to 7, wherein the hydrocarbon feedstock comprises ethane, propane, butanes, light naphtha, heavy naphtha, gas oil, or mixtures thereof.

9. A method for inhibiting the formation and deposition of pyrolytic coke on the heated metallic surfaces in contact with a hydrocarbon feedstock which is undergoing pyrolytic processing to produce lower hydrocarbon fractions and the metallic surfaces have a temperature of about 760°C (1400°F) or higher, which comprises adding to the hydrocarbon feedstock being pyrolytically processed a synergistic combination of a boron compound and a dihydroxybenzene compound.

10. A method according to claim 9, wherein the boron compound is selected from an ammonium borate, boron oxide and sodium borate.

11. A method according to claim 10, wherein the ammonium borate is ammonium biborate or ammonium pentaborate.

12. A method according to any of claims 9 to 11, wherein the dihydroxybenzene compound is selected from hydroquinone, resorcinol, catechol, 1,2-naphthoquinone, and 4-tert-butyl resorcinol.

13. A method according to any of claims 9 to 12, wherein the weight ratio of the dihydroxy compound to the boron compound is from 1:1 to 4:1.

14. A method according to any of claims 9 to 13, wherein the boron compound is contained in a glycollic carrier.

15. A method according to claim 14, wherein the glycollic carrier is ethylene glycol.

16. A method according to any of claims 9 to 15, wherein the dihydroxybenzene compound is contained in a water:butyl carbitol co-solvent carrier or a water:ethylene glycol co-solvent carrier.

17. A method according to any of claims 9 to 16, wherein the compound is added to the hydrocarbon feedstock from about 1 ppm to about 2500 ppm per million parts of hydrocarbon feedstock.

18. A method according to any of claims 9 to 17, wherein the dihydroxybenzene compound is added to the hydrocarbon feedstock from about 1 part per million to about 2500 parts per million per million parts of hydrocarbon feedstock.

19. A method according to any of claims 9 to 18, wherein the hydrocarbon feedstock is ethane, propane, butanes, light naphtha, heavy naphtha, gas oil or mixtures thereof.

20. A method according to any of claims 9 to 19, wherein the metallic surface is a ferrous metal.

21. A composition for inhibiting the formation and deposition of pyrolytic coke which comprises a boron compound and a dihydroxybenzene compound.

22. A composition according to claim 21, wherein the boron compound is selected from an ammonium borate, boron oxide and sodium borate.

23. A composition according to claim 22, wherein the ammonium borate is ammonium biborate or ammonium pentaborate.

24. A composition according to any of claims 21 to 23, wherein the dihydroxybenzene compound is selected from hydroquinone, resorcinol, catechol, 1,2-naphthoquinone, 1,4-naphthoquinone, and 4-tert-butyl resorcinol.

25. A composition according to any of claims 21 to 24, wherein the weight ratio of the dihydroxybenzene compound to the boron compound is from 1:1 to 4:1.

26. A compoistion according to any of claims 21 to 25, wherein the boron compound is contained in a glycollic carrier.

27. A composition according to claim 26, wherein the glycollic carrier is ethylene glycol.

28. A composition according to any of claims 21 to 27, wherein the dihydroxybenzene compound is contained in a water:butyl carbitol co-solvent carrier or a water:ethylene glycol co-solvent carrier.